# EUROPEAN PATENT APPLICATION

(11) **EP 1 449 490 A1**
(43) Date of publication of application: **25.08.2004**
(21) Application number: 03425113.2
(22) Date of filing: 24.02.2003
(51) Int. Cl.: A61B 19/02, B65F 1/00, B65D 5/18

(54) **A protective external casing for refuse containers**

(71) Applicant: Karton S.p.A., 33077 Sacile (PN) (IT)
(72) Inventor: Bressan, Roberto, 33077 Sacile (Pordone) (IT)
(74) Representative: Gotra, Stefano

(57) **Abstract**

The casing is parallelepiped in shape and is destined to contain a bag (2) for refuse collection. The casing is assembled from a flat blank (1) having cut lines and fold lines. The fold lines can be folded to assemble the blank (1) into the shape of the casing. The fold lines define various parts (11, 12, 12', 12", 13, 13', 13", 14, 14', 14", 15, 15') of the blank (1), which will define a bottom wall (50), two first lateral walls (51 and 52), two second lateral walls (53 and 54), and a top wall (55). Only fold lines join a first side (A-B), a second side (B-C), a third side (C-D) and a fourth side (D-A) of the bottom wall (50), as well as an edging of the sides, of a predetermined height and destined to be a part of the lateral walls of the casing.

## Description

The invention is particularly, though not exclusively, useful for use with bag containers destined for refuse collection where the refuse requires special treatment prior to disposal, such as for example hospital refuse, chemical refuse and the like.

In some places, such as hospitals, chemical analysis laboratories, and so on, the refuse produced might be toxic or highly pollutant and has to be specially packaged, differently from normal domestic refuse, and then has to be sent on to special collection centres which follow set procedures for their removal.

In places where dangerous waste materials are produced, special containers are used; in particular moulded rigid containers are used, generally made of plastic, and sack-type soft bags which are then packed in a protective casing, generally made of cardboard or another suitable material. These casings originate as a sheet of cardboard which is then cut to shape and folded.

The present invention relates to the latter-described type of casings.

The advantages of these types of containers, which are used for prevalently solid refuse, relate to their cheapness and the ease with which they can be transported and stored, thanks to the fact that both the sack and the protective casing can be compactly stocked and opened (reaching their final shape) only at moment of use.

A disadvantage which these containers exhibit is that should the refuse contain sharp or pointed objects, the bag, generally made of a film of plastic material, might be slashed open. In these cases, if the refuse, as frequently occurs, contains liquids, these might well dribble out of the bag. With known-type external casings, which are made by folding cut or scored cardboard shapes and assembling the various parts using adhesive tape or the like, these liquids might exit through holes present in the folded parts at the bottom of the covering, even if the material the casing is made of is per se impermeable. Any escape of liquid is absolutely unacceptable and therefore the range of use of bag containers placed inside an external casing is limited, notwithstanding other advantages this type of arrangement might offer.

The main aim of the present invention is to provide a protective casing for refuse containers which ensures perfect a perfect seal of the bottom of the casing.

An advantage of the present invention is that it provides a casing which ensures a bottom seal while at the same time it is easy and economical to produce, transport and store.

A further advantage of the invention is that the casing requires no use of adhesive tapes and the like during assembly.

These aims and advantages and others besides are all attained by the invention as it is characterised in the claims that follow.

Further characteristics and advantages of the present invention will better emerge from the detailed description that follows of a preferred but non-exclusive embodiment of the invention, illustrated purely by way of nonlimiting example in the accompanying figures of the drawings, in which
figure 1 is a plan view of a blank from which the casing will be assembled;
figure 2 is a plan view of the blank of figure 1, folded along a median line;
figure 3 is the blank of figure 2, at a preformed stage of assembly of the casing;
figure 4 is a view of the casing in an open configuration, containing the refuse bag;
figure 5 is a view of the casing of the invention, containing the refuse bag, in a partially closed configuration.

With reference to the figures of the drawings, the protective casing of the invention is destined to contain a bag 2, generally made of a film of plastic material, in which refuse destined for special disposal is collected.

The casing, which when assembled is parallepiped, is assembled from a flat blank 1 made of plastic-coated cardboard or of suitable plastic material, all of known type.

The blank 1 comprises external cut lines which define the outer shape of the blank 1; the blank 1 also contains internal fold or cut lines which when folded will give rise to the casing, i.e. the final parallelepiped shape thereof The fold lines define various parts of the blank 1, denoted in the figures by 11, 12, 12', 13, 13', 13", 14, 14', 14", 15, 15', which, as will be better described herein below, form the bottom wall 50 of the casing, formed by part 11, the two first lateral walls 51 and 52, formed respectively by parts 12 and 12', the two second lateral walls 53 and 54, formed respectively by parts 13, 13', 13" and 14, 14' and 14", and the top wall 55, formed by parts 15, 15'.

The blank 1 comprises two fold lines 1a and 1b, parallel to one another and arranged in the longitudinal direction of the blank 1. The fold lines divide the blank into a first zone, which comprises parts 13, 13' and 13", a second zone, which comprises parts 11,12, 12', and a third zone, which comprises parts 14, 14' and 14". The first, second and third zones are subdivided into the various parts they comprise by two fold lines 1c and 1d, which are parallel to one another, perpendicular to the fold lines 1a and 1b and arranged on opposite sides with respect to the halfway line of the blank 1.

Two further fold lines 1e and 1f are provided parallel to the fold lines 1c and 1d and externally thereof and on opposite sides with respect to the median line of the blank 1. The two fold lines 1e and 1f delimit parts 15 and 15' destined to become the top wall 55 of the casing; parts 15 and 15' are cut, with cuts perpendicular to the fold lines 1e and 1f, so as to enable, as occurs in casings of known type, formation of the top wall which will then be used as a lid to close the casing.

The blank 1 further comprises a fold line 2a which is parallel to the fold lines 1c, 1d, 1e and 1f and which is arranged on the median line of the bottom wall 50, i.e. on the median line of the blank 1 (which will be described in more detail herein below).

The first and third zone of the blank 1 exhibit a height which is at least equal to half the width of the second lateral walls. In particular, the parts of these zones which are arranged on a side of the fold line 2a exhibit a width which is equal to half the second lateral walls, while the parts of the zones located at the other side of the fold line 2a exhibit a slightly greater width so as to define, on these second parts, two wings 3 and 4 which, when the blank 1 is folded, as will be better described herein below, along the fold line 2a, are superposed on the parts of the first and third zones located on the other side of the fold line 2a and can be solidly connected thereto. Although this conformation is extremely handy and functional, if so desired the first and third zones of the blank 1 can be of constant heights equal to half the width of the second lateral walls; in this case, as will better emerge herein below, the formation of the second lateral walls is slightly different.

A, B, C and D denote the comers of the bottom wall 50 of the blank 1, which bottom wall 50 is in fact also part 11 of the blank 1. The bottom wall 50 is delimited by a first side A-B, a second side B-C, a third side C-D and a fourth side D-A. The above-described conformation of the blank 1 and its fold and cut lines is such that in the first side A-B, the second side B-C, third side C-D and the fourth side D-A of the bottom wall 50 of the casing, and up to a predetermined height of the four sides destined to be part of the lateral walls of the casing, only fold lines are present. The predetermined height is at least equal to half the width of the second lateral walls, i.e. at least the same as the width of the parts 13" and 14" of the first and second zones of the blank 1 which are adjacent to sides A-B and C-D of the bottom wall (at parts 12 and 12' arranged adjacent to sides A-C and B-D of the bottom wall the height is greater). This detail is extremely important for ensuring, as will be seen herein below, the seal of the bottom of the casing. In part 13" of the first zone, which is connected to side A-B of the bottom wall 50, there are two inclined fold lines 3a and 3b, which with side A-B form an isosceles triangle. These inclined fold lines start respectively from comers A and B and meet where line 2a meets the external cut line of the blank 1. Similarly, in part 14" of the third zone, which is connected to the side C-D of the bottom wall 50, there are two inclined fold lines 4a and 4b, which with side C-D form an isosceles triangle. These inclined fold lines start respectively from comers C and D and meet where line 2a meets the external cut line of the blank 1.

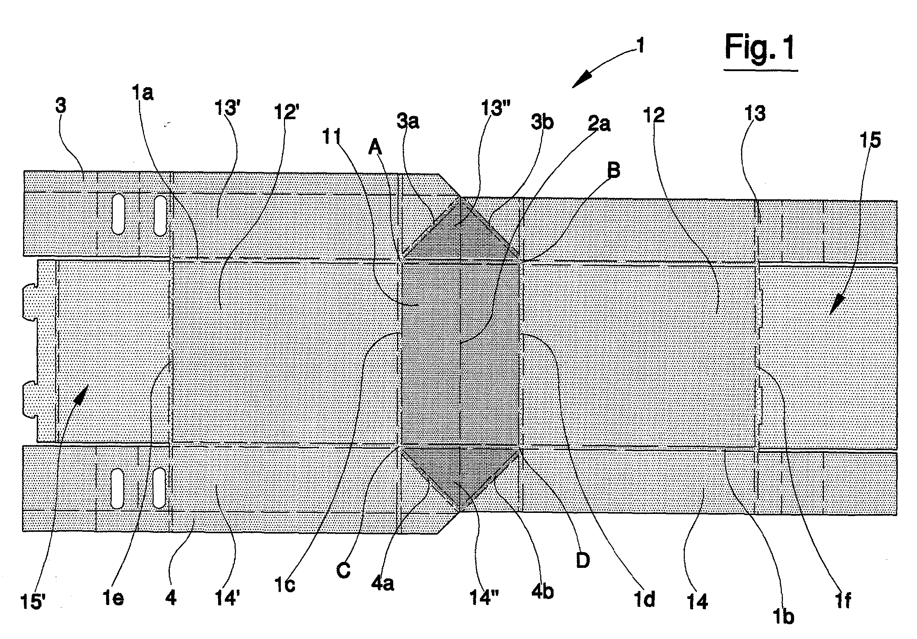

On the external side (which, looking at figure 1 is the non-visible side) of each of the two wings 3 and 4, a strip of glue 30 and 31 is provided in the zone of the wings close to the fold lines 2a and 3a. The strip of glue 30 and 31 is of about the same length as twice the height of the parts 13" and 14", and is protected with a sheet of detachable material. In an embodiment not including the wings 3 and 4, the glue strips can be put on the parts destined to constitute the second walls of the casing.

The strips can be made using normal covered bi-adhesive tapes, a sheet of silicone-treated paper, or in any case known-type glues and strips.

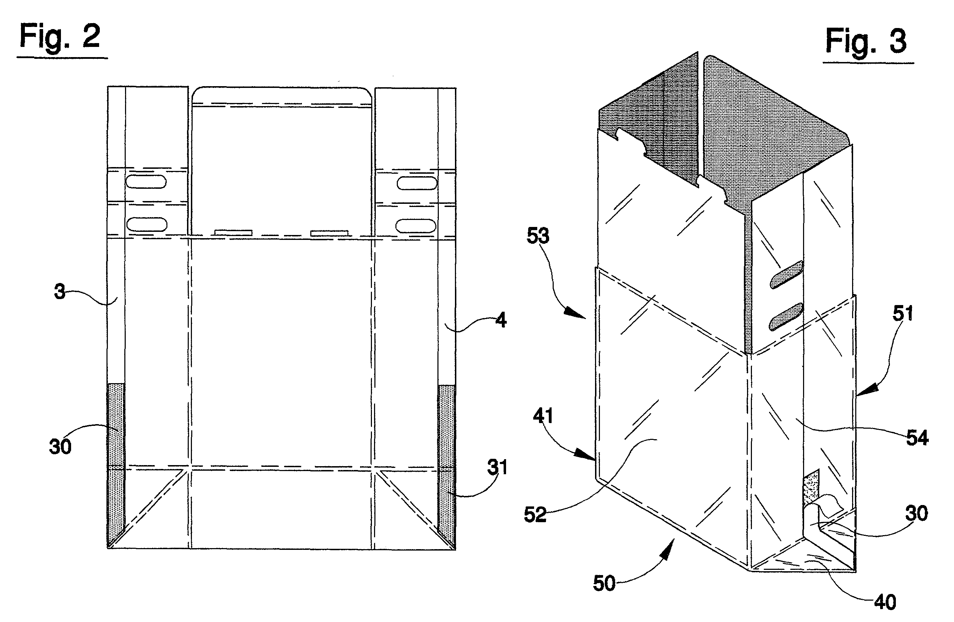

Starting from a flat sheet of suitable material, the cuts and scores are made as above-described, so as to obtain the blank 1 illustrated in figure 1. Subsequently the blank 1 is folded along the median fold line 2a so that the two halves of the blank 1 are laid one above the other. The wings 3 and 4 are then glued using heat-welding, glues or any other suitable known systems for realising sealed joints, on the opposite surfaces of the blank 1. In an embodiment not including the wings 3 and 4, the side-by-side parts of the second walls can be joined by head-to-head heat welding or by adhesive tape. Thus the flat structure of figure 2 is obtained; it is a sort of envelope, open at the top and hermetically sealed at all other sides. The structure, in which the glue strips 30 and 31 are still protected by the relative sheets, is very compact and can easily be stored, or sent on to a user and stored by him or her.

At moment of use, the structure is "opened" by straightening the fold 2a and folding folds 1c and 1d in order to reconstruct the flat bottom wall 50 of the casing. Triangles 40 and 41, which form laterally by the side of the second lateral walls following the folding of the inclined fold lines 3a, 3b, 4a and 4b (see figure 3), after having detached the protective sheets from the glue strips 30 and 31, are folded upwards so that the part of each strip present on each triangle meets and is glued to the remaining part of strip present on the respective second lateral surfaces.

In this way a parallelepiped casing is obtained, open at the top, in which the bottom, to a height corresponding to the height of the triangles 40 and 41, i.e. equal to half the width of the second walls of the casing, exhibits no opening whatsoever.

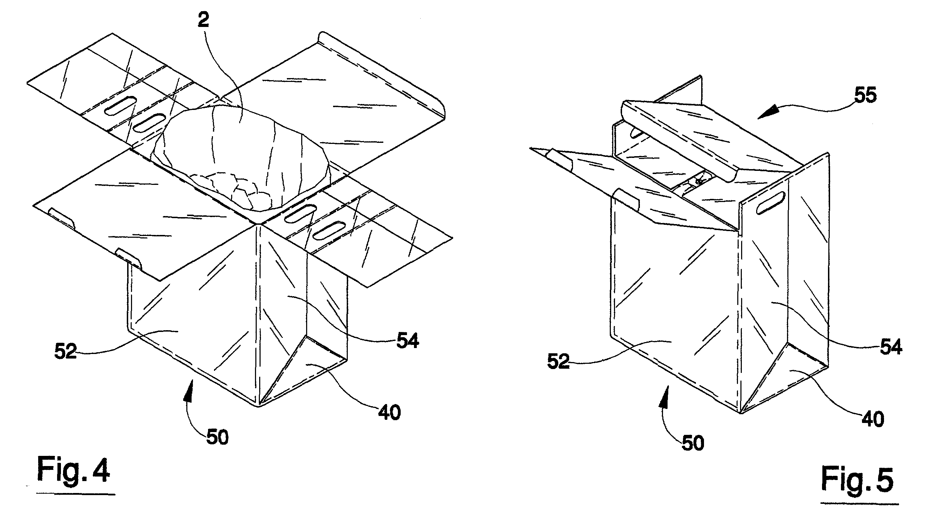

When the fold lines 1e and 1f are folded, the varies parts making up the top part of the casing are opened and the bag 2 can be installed. The refuse will go in the bag (figure 4). However, the bag could be inserted and possibly fixed inside the casing by the manufacturer. In that case, when the casing is opened up by the user, the bag will already be in position inside the casing.

It is worth noting that the shapes of the various parts making up the top part of the casing are not peculiar to the invention and could be different from those illustrated. The illustrated systems, however, enable the casing to be closed with no use of adhesive tapes or the like.

Once the bag has been filled with refuse and closed, the casing is also closed, by folding and jointing the various parts which make up the top part (figure 5), and is ready to be sent on for disposal.

With the invention, any lacerations in the bag which might occur during the introduction of refuse or during transport of the closed casing, and which might occasion loss of fluid or other materials (for example powders) from the bag, would not lead to problems in that the bottom of the casing, to a height equal to half the width of the second walls, is hermetically sealed. The only part where an opening could occur is at the vertex of the triangles 40 and 41, which for this very reason is located at a height equal to half the width of the second walls.

## Claims

1. A protective external casing for refuse containers, of a parallelepiped shape, destined to contain a bag (2) for collecting refuse, realized from a flat blank (1) comprising external cut lines, which define a shape of a flat development of the blank 1, and internal fold or cut lines, which can be folded to convert the blank (1) into a casing, a shape of which casing is defined by various parts (11, 12, 12', 12", 13, 13', 13", 14, 14', 14", 15, 15') of the blank (1) which various parts are destined to define a bottom wall (50), two first lateral walls (51 and 52), two second lateral walls (53 and 54) and a top wall (55) of the casing, **characterised in that** only folded lines join a first side (A-B), a second side (B-C), a third side (C-D) and a fourth side (D-A) of the bottom wall 50 of the casing, and a surround of the four sides, destined to constitute a part of the lateral walls of the casing, of a predetermined height.

2. The casing of claim 1, **characterised in that**: the blank (1) comprises two fold lines (1a and 1b) which divide the blank (1) into a first zone, comprising a first plurality of parts (13, 13', 13"), a second zone, comprising a second plurality of parts (11, 12, 12'), and a third zone, comprising a third plurality of parts (14, 14', 14"); the second zone defining the bottom wall (50) and the two first lateral walls (51 and 52), and the first and third zone defining the two second lateral walls (53 and 54); parts composing each zone being divided by two fold lines (1c and 1d); a part (13") of the first plurality of parts of the first zone being connected to a side (A-B) of the bottom wall (50) and exhibiting two inclined fold lines (3a and 3b) which together with the side (A-B) form an isosceles triangle; a part (14") of the plurality of parts of the third zone being connected to the side (C-D) of the bottom wall (50) and exhibiting two inclined fold lines (4a and 4b) which together with the side (C-D) form an isosceles triangle.

3. The casing of claim 2, **characterised in that**: the blank (1) comprises a fold line (2a), parallel to the fold lines (1c and 1d), arranged on the median line of the bottom wall (50); the first and third zones of the blank (1) exhibiting a height which is equal to at least a half of a width of the second lateral walls (53 and 54); parts of the zones located on a side of the fold line (2a) exhibiting a width which is slightly greater than a width of parts of the zones located on the side of the fold line (2a), so as to define two wings (3 and 4) which, when the blank (1) is folded along the fold line (2a) are superposed on the parts of the first and third zones located on the other side of the fold line (2a) not having wings, and can be solidly constrained thereto.

4. The casing of claim 3, **characterised in that** a strip of glue (30, 31), protected by a sheet of detachable material, is attached on an external side of each of the two wings (3 and 4) and close to the fold line (2a); the strip of glue (30, 31) being of about double a height of the parts (13", 14").

5. The casing of claim 1, **characterised in that** a minimum height of the surrounding of the sides of the bottom wall (50) of the casing, destined to constitute a part of the lateral walls of the casing, which meet only along fold lines, is equal to a width of the second lateral walls of the casing.
